# EUROPEAN PATENT APPLICATION

(11) **EP 1 413 574 A1**
(43) Date of publication of application: **28.04.2004**
(21) Application number: 02743893.6
(22) Date of filing: 09.07.2002
(51) Int. Cl.: C07D 207/12

(54) **PYRROLIDINE DERIVATIVES AND METHOD OF SYNTHESIZING THESE**

(30) Priority: 27.07.2001 JP 2001227011
(71) Applicant: Eizai Co., Ltd., Bunky-ku, Tokyo 112-0002 (JP); Chiba, Hiroyuki, Bunkyo-ku, Tokyo 112-0013 (JP); Nakamura, Taiju, Kashima-gun, Ibaraki 314-0144 (JP)
(72) Inventor: TAGAMI, Katsuya, Tsuchiura-shi, Ibaraki 300-0845 (JP); CHIBA, H., Room 1202, Fujiwa-city-homes-bunkyo-ot, Tokyo 112-0013 (JP); NAKAMURA, T., Room 204, Grand-chariot 2bankan, Kashima-gun, Ibaraki 344-0144 (JP); KUBOTA, Manabu, Tsukuba-shi, Ibaraki 305-0005 (JP); MATSUI, Makoto, Room 305, Tsukuba-shi, Ibaraki 305-0051 (JP); KAYANO, Akio, Kashima-shi, Ibaraki 314-0006 (JP); MIYAZAWA, Mamoru, Kashima-gun, Ibaraki 314-0144 (JP); ABE, Taichi, Narita-shi, Chiba 286-0048 (JP)
(74) Representative: Jones, Helen Marjorie Meredith
(86) International application number: PCT/JP2002/006967
(87) International publication number: WO 2003/011825

(57) **Abstract**

The object of the invention is to provide a process for efficient, inexpensive and stereoselective production of (2S,4S)-2-[[(3R)-pyrrolidin-3-yl-(R)-hydroxy] methyl] pyrrolidin-4-thiol dihydrochloride useful as an intermediate in production of novel carbapenem, and the intermediate is produced via a novel crystallizable intermediate (2S,4R)-N-t-butoxycarbonyl-2-[[(3R)-N-t-butoxycarbonyl pyrrolidin-3-yl-(R)-hydroxy]methyl]-4-hydroxypyrrolidine from a (2S,4R)-4-alkylsilyloxy-N-t-butoxycarbonylpyridine-2-carbal dehyde derivative by aldol reaction using an asymmetric assistant such as amino-alcohol.

## Description

### TECHNICAL FIELD

The present invention relates to an intermediate useful for production of a novel carbapenem derivative useful as an antimicrobial agent and a method of producing the same.

### BACKGROUND ART

A method of synthesizing the objective compound in the present invention, that is, (2S,4S)-2-[[(3R)-pyrrolidin-3-yl-(R)-hydroxy] methyl] pyrrolidin-4-thiol, is shown in JP-A 8-73462 and JP-A 11-35556.

Study on synthesis of a large number of compounds with respect to carbapenem antibiotics has been conducted since thienamycin separated from Streptomyces catleya was found in 1976.

As a result of extensive study, the present inventors found that carbapenem derivatives having a 2-substituted pyrrolidine-4-thio group at the 2-position possess an excellent antimicrobial action, and they filed a patent application therefor in recent years (JP-A 8-73462). Among these derivatives, carbapenem derivatives having optically active (2S,4S)-2-[[(3R)-pyrrolidin-3-yl-(R)-hydroxy]methyl]pyrroli dine-4-thiol are very useful because of their excellent antimicrobial action.

The present inventors also found a useful method of synthesizing optically active (2S,4S)-2-[[(3R)-pyrrolidin-3-yl-(R)-hydroxy] methyl] pyrrolidin-4-thiol which is a useful intermediate in synthesis of the carbapenem derivatives, and filed patent applications therefor (JP-A 8-73462 and JP-A 11-35556). However, the method described in JP-A 8-73462 supra is disadvantageous in that because diastereomers of unnecessary configuration are also formed in the first step, the unnecessary diastereomers should be separated and removed, and the yield of diastereomers of necessary configuration is as low as 30.2%. Accordingly, the invention described in JP-A 11-35556 supra employed a stereoselective production method (Evans method), thus significantly increasing the yield of the desired product to 63 to 67%. In this method, however, the production of the desired compound in industrial scale is difficult because of the production thereof via an explosive azide compound as an intermediate and from an economical viewpoint of reagents used.

In view of these problems in the above art, a main object of the present invention is to provide a useful process of synthesizing optically active (2S,4S)-2-[[(3R)-pyrrolidin-3-yl-(R)-hydroxy] methyl] pyrrolidin-4-thiol with improvements in selectivity, efficiency, safety and purification.

### DISCLOSURE OF INVENTION

In the study of a novel process of synthesizing ((2S,4S)-2-[[(3R)-pyrrolidin-3-yl-(R)-hydroxy]methyl]pyrrol idin-4-thiol dihydrochloride) useful in production of carbapenem derivatives, the present inventors invented a process for producing the compound via a novel substance that is, ((2S,4R)-N-t-butoxycarbonyl-2-[[(3R)-N-t-butoxycarbonylpyrr olidin-3-yl-(R)-hydroxy] methyl]-4-t-hydroxypyrrolidine) as an intermediate which is very excellent in crystallizability, is stable and enables efficient separation of unnecessary diastereomers as byproducts.

That is, the present invention is as follows:

### (1) A process for producing an optically active pyrrolidine-4-thiol derivative (IX) represented by the following formula, or salts thereof or hydrates thereof:

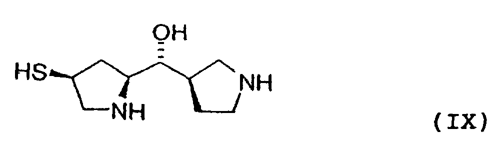

which comprises introducing a protecting group into a hydroxyl group of a pyrrolidine derivative (I) represented by the formula: 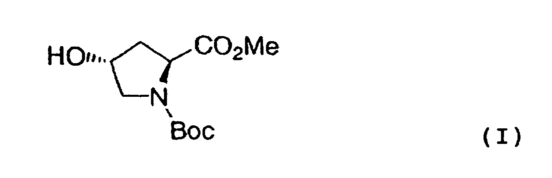 wherein Boc represents a t-butoxycarbonyl group, to form a pyrrolidine-2-carboxylate derivative (II) represented by the formula: 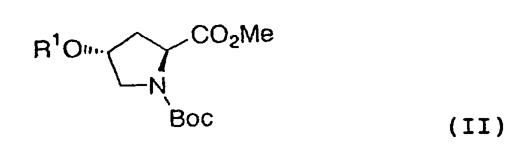 wherein R¹ represents a hydrogen atom or a tri(C₁₋₆ alkyl)silyl group, and Boc has the same meaning as defined above,
then reducing an alkoxycarbonyl group of the compound (II) to give a pyrrolidine-2-carbaldehyde (III) represented by the formula: 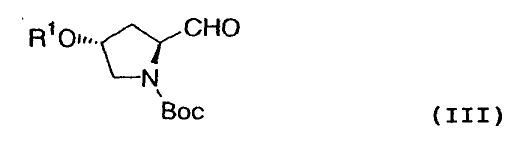 wherein R¹ and Boc have the same meaning as defined above,
then subjecting the compound (III) to asymmetric aldol reaction with a pyrrolidone derivative (V) represented by the formula: 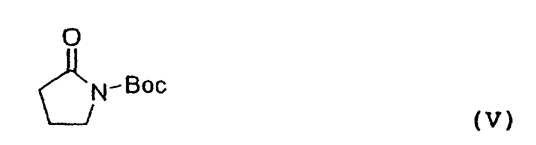 wherein Boc has the same meaning as defined above, by using as an asymmetric assistant an optically active aminoethanol derivative (IVa) represented by the formula: 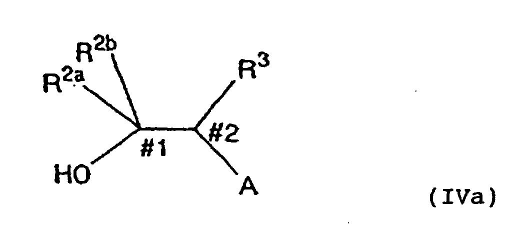 wherein both / either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group) , an 1-pyrrolidinyl group, a 4-morpholinyl group or a 2-isoindolinyl group, an optically active pyrrolidine derivative (IVb) represented by the formula: 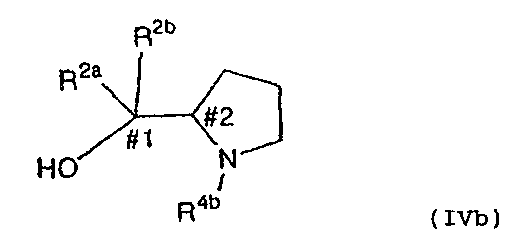 wherein #1, #2, R^{2a}, R^{2b} and R^{4b} have the same meaning as defined above, optically active cinchonidine and/or (2R,3S)-3-dimethylaminoborneol, thereby forming an optically active pyrrolidine derivative (VI) represented by the formula: 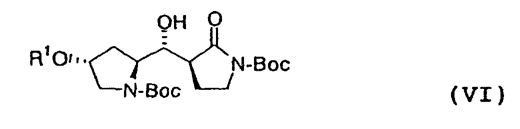 wherein R¹ and Boc have the same meaning as defined above,
then reducing the optically active pyrrolidine derivative (VI) to form a pyrrolidine derivative (VII) represented by the formula: 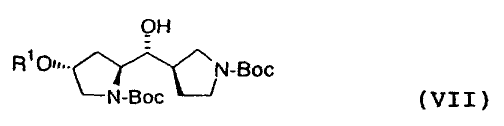 wherein R¹ and Boc have the same meaning as defined above,
then selectively eliminating an alkylsilyl group from the compound (VII) to form an optically active 4-hydroxypyrrolidine derivative (VIII) represented by the formula: 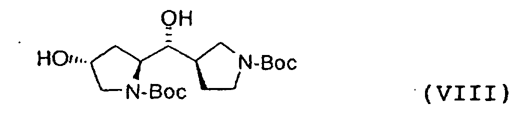 wherein Boc has the same meaning as defined above, and
then converting a hydroxyl group of the compound (VIII) into an acylthio group, to eliminate the protecting group,
whereby the optically active pyrrolidine-4-thiol derivative (IX), or salts thereof or hydrates thereof are obtained.

### (2) A process for producing an optically active pyrrolidine derivative (VI) represented by the following formula, or hydrates thereof:

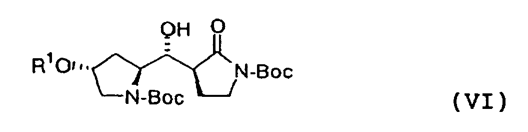

wherein R¹ has the same meaning as defined above and Boc represents a t-butoxycarbonyl group,
which comprises introducing a protecting group into a hydroxyl group of a pyrrolidine derivative (I) represented by the formula: 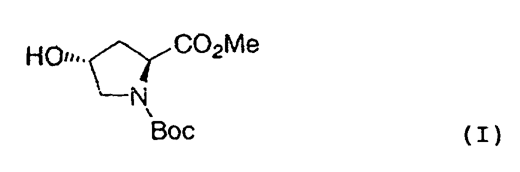 wherein Boc has the same meaning as defined above, to form a pyrrolidine-2-carboxylate derivative (II) represented by the formula: 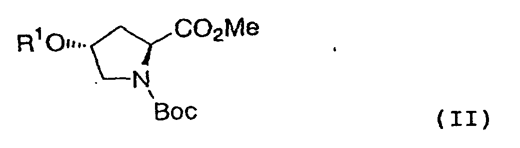 wherein R¹ and Boc have the same meaning as defined above,
then reducing an alkoxycarbonyl group of the compound (II) to give a pyrrolidine-2-carbaldehyde derivative (III) represented by the formula: 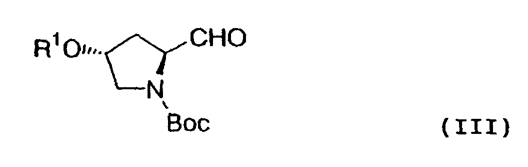 wherein R¹ and Boc have the same meaning as defined above, and
then subjecting the compound (III) to asymmetric aldol reaction with a pyrrolidone derivative (V) represented by the formula: 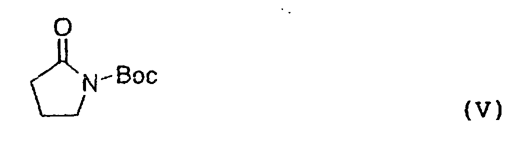 wherein Boc has the same meaning as defined above, by using as an asymmetric auxiliary, an optically active aminoethanol derivative (IVa) represented by the formula: 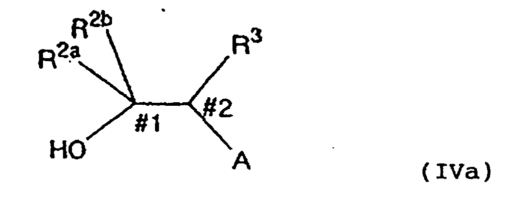 wherein both / either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group) , an 1-pyrrolidinyl group, a 4-morpholinyl group or a 2-isoindolinyl group, an optically active pyrrolidine derivative (IVb) represented by the formula: 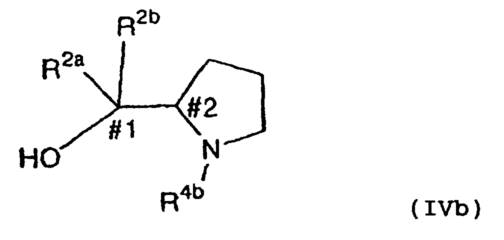 wherein #1, #2, R^{2a}, R^{2b} and R^{4b} have the same meaning as defined above, optically active cinchonidine and/or (2R,3S)-3-dimethylaminoborneol,
whereby the optically active pyrrolidine derivative (VI) or hydrates thereof are obtained.

### (3) A process for producing an optically active pyrrolidine-4-thiol derivative (IX) represented by the following formula, or salts thereof or hydrates thereof:

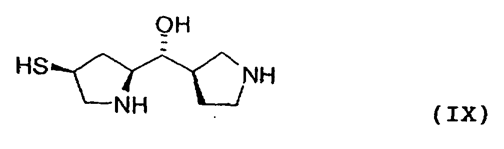

which comprises reducing an optically active pyrrolidine derivative (VI) represented by the formula: 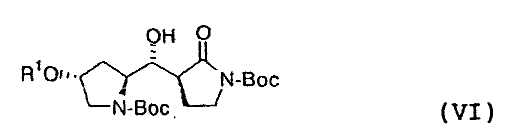 wherein R¹ and Boc have the same meaning as defined above, to form a pyrrolidine derivative (VII) represented by the formula: 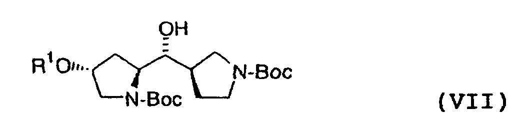 wherein R¹ and Boc have the same meaning as defined above,
then selectively eliminating an alkylsilyl group from the compound (VII), to form an optically active 4-hydroxypyrrolidine derivative (VIII) represented by the formula: 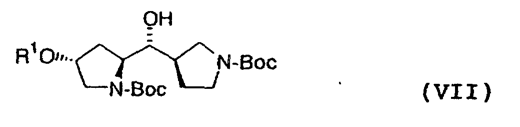 wherein Boc has the same meaning as defined above, and
then converting a hydroxyl group of the compound (VIII) into an acylthio group to eliminate the protective group,
whereby the optically active pyrrolidine-4-thiol derivative (IX), or salts thereof or hydrates thereof are obtained.

### (4) A process for producing an optically active 4-hydroxypyrrolidine derivative (VIII) represented by the following formula, or hydrates thereof:

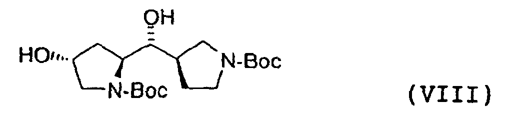

wherein Boc has the same meaning as defined above,
which comprises subjecting a pyrrolidone compound (V) represented by the formula: 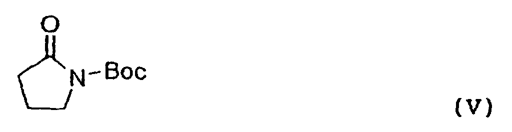 wherein Boc has the same meaning as defined above, to asymmetric aldol reaction with a pyrrolidine-2-carbaldehyde derivative (III) represented by the formula: 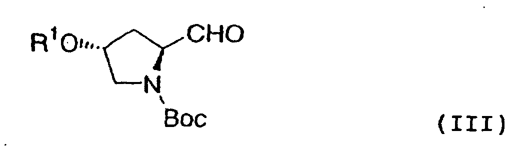 wherein R¹ and Boc have the same meaning as defined above, by using as an asymmetric assistant an opticallyactive aminoethanol derivative (IVa) represented by the formula: 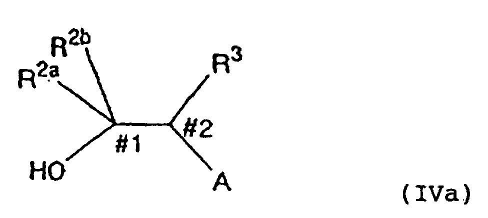 wherein both /either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group) , an 1-pyrrolidinyl group, a 4-morpholinyl group or a 2-isoindolinyl group, an optically active pyrrolidine derivative (IVb) represented by the formula: 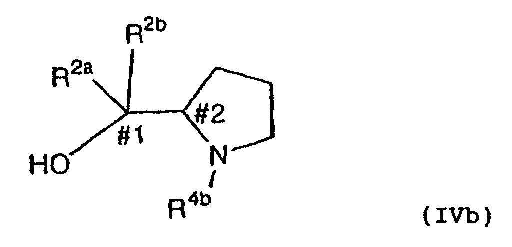 wherein #1, #2, R^{2a}, R^{2b} and R^{4b} have the same meaning as defined above, optically active cinchonidine and/or (2R,3S)-3-dimethylaminoborneol, thus obtaining an optically active pyrrolidine derivative (VI) represented by the formula (VI): 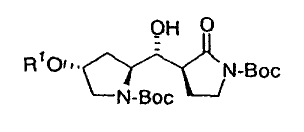 wherein R¹ and Boc have the same meaning as defined above,
then reducing the optically active pyrrolidine derivative (VI) to form a pyrrolidine derivative (VII) represented by the formula: 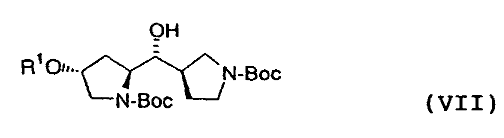 wherein R¹ and Boc have the same meaning as defined above, and then selectively eliminating an alkylsilyl group from the compound (VII),
whereby the optically active 4-hydroxypyrrolidine derivative (VIII) or hydrates thereof are obtained.

### (5) A process for producing an optically active pyrrolidine derivative (VI) represented by the following formula, orhydrates thereof:

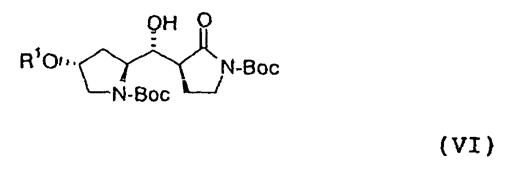

wherein R¹ and Boc have the same meaning as defined above,
which comprises subjecting a pyrrolidine derivative (V) represented by the formula: 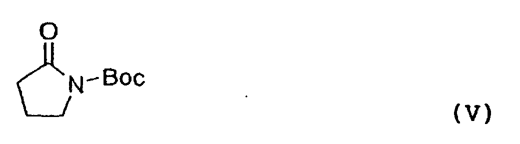 wherein Boc has the same meaning as defined above, to asymmetric aldol reaction with a pyrrolidine-2-carbaldehyde derivative (III) represented by the formula: 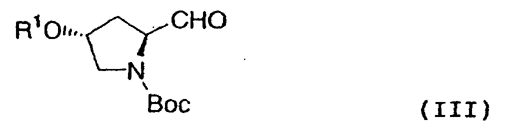 wherein R¹ and Boc have the same meaning as defined above, by using an optically active aminoethanol derivative (IVa) represented by the formula: 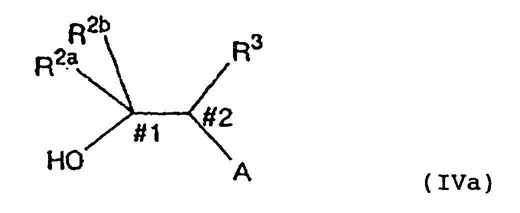 wherein both / either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group), an 1-pyrrolidinyl group, a 4-morpholinyl group or a 2-isoindolinyl group, an optically active pyrrolidine derivative (IVb) represented by the formula: 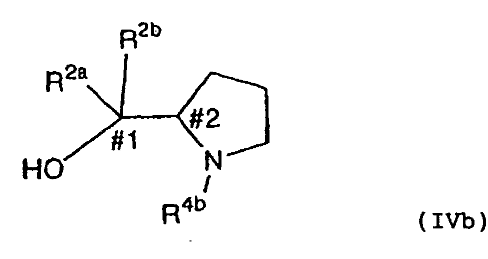 wherein #1, #2, R^{2a}, R^{2b} and R^{4b} have the same meaning as defined above, optically active cinchonidine and/or
(2R,3S)-3-dimethylaminoborneol,
whereby the optically active pyrrolidine derivative (VI) or hydrates thereof are obtained.

### (6) A process for producing an optically active 4-hydroxypyrrolidine derivative (VIII) represented by the following formula, or hydrates thereof:

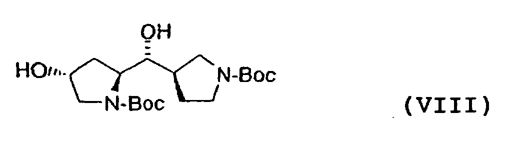

wherein Boc has the same meaning as defined above, which comprises selectively eliminating an alkylsilyl group from a pyrrolidine derivative (VII) represented by the formula: 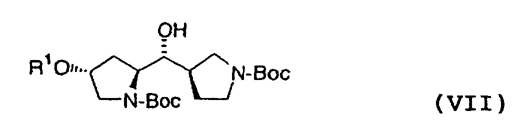 wherein R¹ and Boc have the same meaning as defined above.

### (7) The process according to any one of the above items (1), (2), (4) and (5), wherein the optically active asymmetric assistant is (R)-1-amino-2-benzyl-1,3-diphenyl-2-propanol.

### (8) The process according to any one of the above item (1), (2), (4) and (5), wherein the optically active asymmetric assistant is (R)-3-amino-2-benzyl-1,4-diphenyl-2-butanol.

### (9) An optically active pyrrolidine derivative (VII) represented by the following formula, or hydrates thereof:

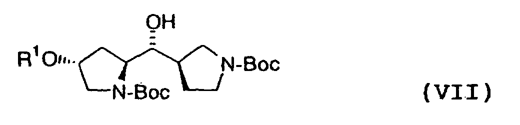

wherein R¹ and Boc have the same meaning as defined above.

The structural formula of the compound in the present specification may, for convenience' sake, indicate a certain isomer, but this invention encompasses all possible isomers which may occur in the structures of the compound, for example geometric isomer, optical isomer based on asymmetrical carbon, stereoisomer and tautomer, as well as a mixture of such isomers, and the compound of the invention is not limited to the formula shown for convenience' sake. All salts of the compound or hydrates thereof also fall under the scope of the invention.

The salts in the present invention are not particularly limited, and examples of such salts include salts of inorganic acids, such as hydrof luoride, hydrochloride, sulfate, nitrate, perchlorate, phosphate, carbonate, bicarbonate, hydrobromate, hydroiodate etc.; addition salts of organic carboxylic acids, such as acetate, maleate, fumarate, oxalate, lactate, tartrate, trifluoroacetete etc.; addition salts of organic sulfonic acid, such as methane sulfonate, trifluoromethane sulfonate, ethane sulfonate, hydroxymethane sulfonate, hydroxyethane sulfonate, benzene sulfonate, toluene sulfonate, taurine salt, etc.; addition salts of amines, such as trimethylamine salt, triethylamine salt, pyridine salt, procaine salt, picoline salt, dicyclohexylamine salt, N,N-dibenzylethylene diamine salt, N-methyl glucamine salt, diethanol amine salt, triethanol amine salt, tris(hydroxymethylamino)methane salt, phenethyl benzyl amine salt etc.; addition salts of alkali metals, such as sodium salt, potassium salt etc.; salts of alkaline earth metals, such as magnesium salt, calcium salt etc.; and salts of amino acids, such as arginine salt, lysine salt, serine salt, glycine salt, aspartate, glutamate, etc. These salts are preferably pharmacologically acceptable salts.

In the present invention, the "C₁₋₆ alkyl group" represented by R¹, R², R³ and R⁴ refers to a linear or branched alkyl group containing 1 to 6 carbon groups, and specifically it is for example a methyl group, ethyl group, n-propyl group, i-propyl group, n-butyl group, i-butyl group, sec-butyl group, t-butyl group, n-pentyl group, i-pentyl group, sec-pentyl group, t-pentyl group, neopentyl group, 1-methylpentyl group, 2-methylpentyl group, 1,1-dimethylpropyl group, 1,2-dimethylpropyl group, n-hexyl group, i-hexyl group, 1-methylpentyl group, 2-methylpentyl group, 3-methylpentyl group, 1,1-dimethylbutyl group, 1,2-dimethylbutyl group, 2,2-dimethylbutyl group, 1,3-dimethylbutyl group, 2,3-dimethylbutyl group, 3,3-dimethylbutyl group, 1-ethylbutyl group, 2-ethylbutyl group, 1,1,2-trimethylpropyl group, 1,2,2-trimethylpropyl group, 1-ethyl-1-methylpropyl group, 1-ethyl-1-methylpropyl group or 1-ethyl-2-methylpropyl group, preferably a methyl group, ethyl group, n-propyl group, i-butyl group, sec-butyl group, t-butyl group, 1-ethyl-2-methylpropyl group or 1-ethyl-2-methylpropyl group, more preferably a methyl group, ethyl group, n-propyl group, i-propyl group, n-butyl group, i-butyl group, sec-butyl group, t-butyl group, n-pentyl group, i-pentyl group, sec-pentyl group, t-pentyl group, neopentyl group, 1-methylbutyl group, 2-methylbutyl group, 1,1-dimethylpropyl group, 1,2-dimethylpropyl group, n-hexyl group or i-hexyl group, still more preferably a methyl group, ethyl group, n-propyl group, i-propyl group, n-butyl group, i-butyl group, sec-butyl group, t-butyl group, n-pentyl group, i-pentyl group, sec-pentyl group, t-pentyl group, neopentyl group, 1-methylbutyl group, 2-methylbutyl group, 1,1-dimethylpropyl group or 1,2-dimethylpropyl group, further more preferably a methyl group, ethyl group, n-propyl group, i-propyl group, n-butyl group, i-butyl group, sec-butyl group or t-butyl group, most preferably a methyl group, ethyl group, n-propyl group or i-propyl group.

In the present specification, the "C₆₋₁₄ aryl group" refers to a C6 to C14 aromatic ring group and specifically it is for example a phenyl group, 1-naphthyl group, 2-naphthyl group, as-indacenyl group, s-indacenyl group or acenaphthylenyl group.

In the present specification, the "C₃₋₈ cycloalkyl group" refers to a C3 to C8 alicyclic hydrocarbon group, and specifically it is for example a cyclopropyl group, cyclobutyl group, cyclopentyl group, cyclohexyl group, cycloheptyl group, cyclopropenyl group, cyclobutenyl group, cyclopentenyl group, cyclohexenyl group or cycloheptenyl group.

In the present specification, the "C₆₋₁₄ aryl-C₁₋₆ alkyl group" refers to a group wherein the above-defined C₁₋₆ alkyl group is substituted with the above-defined C₆₋₁₄ aryl group, and specifically it is for example a benzyl group, phenethyl group, 1-naphthylmethyl group or 2-naphthylmethyl group.

The asymmetric assistant in the present invention includes optically active aminoethanol derivatives, cinchonidine and optically active aminoborneol derivatives. The optically active aminoethanol derivatives include, for example,
(1R,2S)-2-dimethylamino-1,2-diphenylethanol,
(1R,2S)-2-pyrrolidino-1,2-diphenylethanol,
(1R,2S)-2-morpholino-1,2-diphenylethanol,
(1R,2S)-2-(isoindolin-2-yl)-1,2-diphenylethanol,
(1R,2S)-2-dibutylamino-1-phenyl-1-propanol,
(1R,2S)-2-(azetidin-1-yl)-1-phenyl-1-propanol,
(1R,2S)-2-pyrrolidino-1-phenyl-1-propanol,
(1R,2S)-2-morpholino-1-phenyl-1-propanol,
(2R,3R)-2-amino-4-methyl-1-phenyl-3-pentanol,
(1S,2R)-2-dimethylamino-1,2,-diphenylethanol,
(1S,2R)-2-methylamino-1,2-diphenylethanol,
(1S,2R)-2-amino-1,2-diphenylethanol,
(1S,2R)-2-(N-benzyl-N-methylamino)-1,2-diphenylethanol,
(1S,2R)-2-(isoindolin-2-yl)-1,2-diphenylethanol,
(1S,2R)-2-amino-1-cyclopentyl-3-phenyl-1-propanol,
(2S,3R)-2-amino-4-methyl-1-phenyl-3-pentanol,
(1S,2S)-3-methyl-2-dimethylamino-1-phenyl-1-butanol,
(1S,2S)-2-dimethylamino-1,3-diphenyl-1-propanol,
(2S,3S)-4-methyl-2-dimethylamino-1-phenyl-3-pentanol,
(1S,2S)-3-methyl-2-dimethylamino-1-phenyl-1-propanol,
(1S,2S)-1-amino-3-methyl-1-phenyl-2-butanol,
(S)-3-methyl-1-dimethylamino-1-phenyl-2-butanol,
(S)-2-methylamino-1,1,2-triphenylethanol,
(S)-2-dimethyl-1,1,3-triphenyl-1-propanol,
(S)-2-benzyl-3-dimethylamino-1-phenyl-2-butanol,
(S)-2-benzyl-3-dimethylamino-1,3-diphenyl-2-propanol,
(S)-2-benzyl-3-dimethylamino-5-methyl-1-phenyl-2-hexanol,
(S)-2-benzyl-3-dimethylamino-1,4-diphenyl-2-butanol,
(S)-2-benzyl-3-dimethylamino-4-(2-naphthyl)-1-phenyl-2-buta nol,
(R)-1-amino-2-isopropyl-3-methyl-1-phenyl-2-butanol,
(R)-1-amino-2-benzyl-1,3-diphenyl-2-propanol,
(R)-1-amino-2-ethyl-1-phenyl-2-butanol,
(R)-2-amino-1-dicyclopentyl-3-phenyl-1-propanol and
(R)-3-amino-2-benzyl-1,4-diphenyl-2-butanol, preferably
(R)-1-amino-2-benzyl-1,3-diphenyl-2-propanol,
(R)-3-amino-2-benzyl-1,4-diphenyl-2-butanol,
(R)-3-amino-2-benzyl-4-(2-naphthyl)-1-phenyl-2-butanol,
(R)-2-benzyl-3-methylamino-1,4-diphenyl-2-butanol,
(R)-2-methylamino-1,1,3-triphenyl-1-propanol,
(R)-1,1-dinaphthyl-2-pyrrolidine methanol and
(R)-2-amino-1,1-dinaphthyl-3-phenyl propanol.

### BEST MODE FOR CARRYING OUT THE INVENTION

Hereinafter, each step of the production method of the present invention is described in more detail. 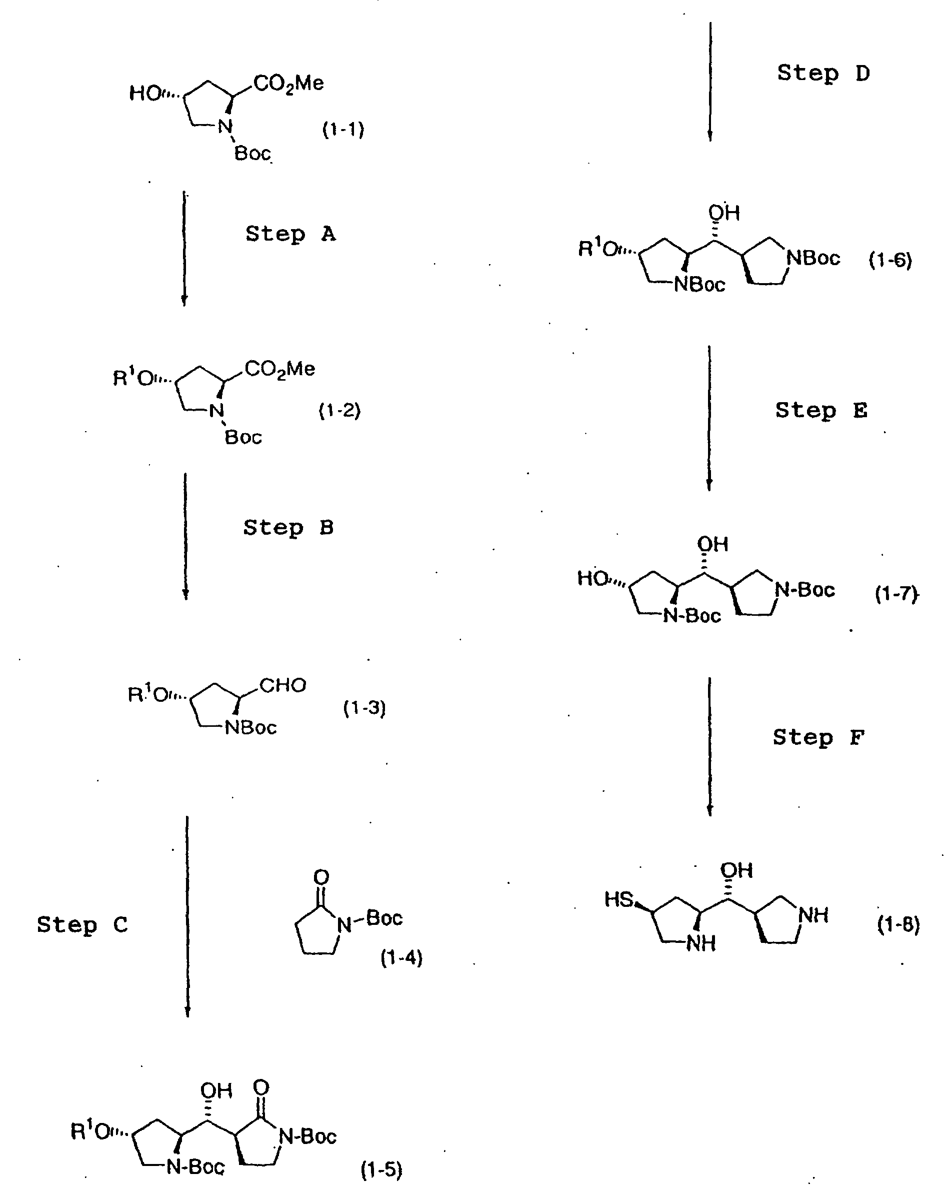 wherein R¹ and Boc each have the same meaning as defined above.

### (Step A)

This step is a step of introducing a protecting group into a hydroxyl group. This step can be carried out under conditions usually used in the reaction of introducing a protecting group of a silylation reagent into a hydroxyl group; for example, a compound represented by the pyrrolidine derivative (1-2) can be obtained by reacting the alcohol compound (1-1) with a silylation reagent such as t-butyldimethylsilyl chloride, t-butyldimethylsilyl trifluoromethane sulfonate, tri-n-butylsilyl chloride, triethylsilyl chloride, trimethylsilyl chloride, tri-n-butylsilyl trifluoromethane sulfonate or trimethylsilyl trifluoromethane sulfonate in the presence of a base such as triethylamine, pyridine or imidazole. As the solvent, tetrahydrofuran, acetonitrile or dimethylformamide can be used, and the reaction is carried out at a temperature of 0°C to -60°C, preferably -20°C to -40°C.

### (Step B)

This step is a step of reducing the ester derivative (1-2) into the aldehyde derivative (1-3). The reaction is carried out by using a base selected from the group consisting of pyrrolidine, morpholine, N-methylpiperidine, piperidine, potassium t-butoxide, sodium phenoxide, and sodium methoxide or a combination of these bases in an arbitrary ratio. The reducing agent includes, for example, sodium bis(2-methoxyethoxy)aluminum hydride. As the solvent, diethyl ether, tetrahydrofuran, dioxane, toluene, 1,2-dimethoxyethane or t-butyl methyl ether can be used. The reaction is carried out at a temperature of 0°C to -78°C, preferably -10°C to -60°C.

### (Step C)

This step is a step of aldol reaction where the aldehyde derivative (1-3) is subjected to condensation reaction (aldol reaction) with the pyrrolidone derivative (1-4). The desired condensate (1-5) can be obtained by condensation reaction of the aldehyde derivative (1-3) with the pyrrolidone derivative (1-4) by using an asymmetric assistant in the presence of a base in a solvent such as diethyl ether, tetrahydrofuran, t-butyl methyl ether or toluene. The reaction is carried out at a temperature of -70°C to -30°C. The base includes n-butyl lithium (n-BuLi), lithium diisopropylamide (LDA), lithium bis(trimethylsilyl)amide (LHMDS), sodium bis(trimethylsilyl)amide (NaHMDS) etc., among which lithium bis(trimethylsilyl)amide (LHMDS) can be preferably used.

The asymmetric assistant includes optically active aminoethanol derivatives, cinchonidine, and optically active aminoborneol derivatives. The optically active aminoethanol derivatives include, for example,
(1R,2S)-2-dimethylamino-1,2-diphenylethanol,
(1R,2S)-2-pyrrolidino-1,2-diphenylethanol,
(1R,2S)-2-morpholino-1,2-diphenylethanol,
(1R,2S)-2-(isoindolin-2-yl)-1,2-diphenylethanol,
(1R,2S)-2-dibutylamino-1-phenyl-1-propanol,
(1R,2S)-2-(azetidin-1-yl)-1-phenyl-1-propanol,
(1R,2S)-2-pyrrolidino-1-phenyl-1-propanol,
(1R,2S)-2-morpholino-1-phenyl-1-propanol,
(2R,3R)-2-amino-4-methyl-1-phenyl-3-pentanol,
(1S,2R)-2-dimethylamino-1,2,-diphenylethanol,
(1S,2R)-2-methylamino-1,2-diphenylethanol,
(1S,2R)-2-amino-1,2-diphenylethanol,
(1S,2R)-2-(N-benzyl-N-methylamino)-1,2-diphenylethanol,
(1S,2R)-2-(isoindolin-2-yl)-1,2-diphenylethanol,
(1S,2R)-2-amino-1-cyclopentyl-3-phenyl-1-propanol,
(2S,3R)-2-amino-4-methyl-1-phenyl-3-pentanol,
(1S,2S)-3-methyl-2-dimethylamino-1-phenyl-1-butanol,
(1S,2S)-2-dimethylamino-1,3-diphenyl-1-propanol,
(2S,3S)-4-methyl-2-dimethylamino-1-phenyl-3-pentanol,
(1S,2S)-3-methyl-2-dimethylamino-1-phenyl-1-propanol,
(1S,2S)-1-amino-3-methyl-1-phenyl-2-butanol,
(S)-3-methyl-1-dimethylamino-1-phenyl-2-butanol,
(S)-2-methylamino-1,1,2-triphenylethanol,
(S)-2-dimethyl-1,1,3-triphenyl-1-propanol,
(S)-2-benzyl-3-dimethylamino-1-phenyl-2-butanol,
(S)-2-benzyl-3-dimethylamino-1,3-diphenyl-2-propanol,
(S)-2-benzyl-3-dimethylamino-5-methyl-1-phenyl-2-hexanol,
(S)-2-benzyl-3-dimethylamino-1,4-diphenyl-2-butanol,
(S)-2-benzyl-3-dimethylamino-4-(2-naphthyl)-1-phenyl-2-butanol,
(R)-1-amino-2-isopropyl-3-methyl-1-phenyl-2-butanol,
(R)-1-amino-2-benzyl-1,3-diphenyl-2-propanol,
(R)-1-amino-2-ethyl-1-phenyl-2-butanol,
(R)-2-amino-1-dicyclopentyl-3-phenyl-1-propanol and
(R)-3-amino-2-benzyl-1,4-diphenyl-2-butanol, preferably
(R)-1-amino-2-benzyl-1,3-diphenyl-2-propanol,
(R)-3-amino-2-benzyl-1,4-diphenyl-2-butanol,
(R)-3-amino-2-benzyl-4-(2-naphthyl)-1-phenyl-2-butanol,
(R)-2-benzyl-3-methylamino-1,4-diphenyl-2-butanol,
(R)-2-methylamino-1,1,3-triphenyl-1-propanol,
(R)-1,1-dinaphthyl-2-pyrrolidine methanol and
(R)-2-amino-1,1-dinaphthyl-3-phenyl propanol.

The reaction is carried at a temperature of 0°C to -78°C, preferably -40°C to -78°C.

### (Step D)

This step is a step of reducing the lactam derivative (1-5) into the amine derivative (1-6). The reaction is carried out by using a reducing agent such as lithium aluminum hydride (LiAlH₄), diisobutyl aluminum hydroxide (DIBAL-H), sodium borohydride (NaBH₄), borane (BH₃), a borane/tetrahydrofuran complex (BH₃·THF), or a borane/dimethylsulfide complex (BH₃·Me₂S) in the presence or absence of an acid (proton acid such as hydrochloric acid, sulfuric acid or trifluoroacetic acid or Lewis acid such as BF₃Et₂O, or iodine) in a reaction solvent such as tetrahydrofuran or dimethoxyethane. The reaction is carried out at 0°C to reflux temperature.

### (Step E)

This step is a step of deprotecting the hydroxyl-protecting group. This reaction can be carried out under conditions usually used in the reaction of deprotecting a protecting group, whereby the hydroxyl group protected with a silyl group is deprotected into a hydroxyl group. Specifically, the reaction can be carried out in the presence of an acid such as hydrogen fluoride, hydrochloric acid, sulfuric acid or phosphoric acid and in the presence of tetrabutyl ammonium fluoride, hydrogen fluoride pyridine, a hydrogen fluoride/triethylamine complex, potassium fluoride, acidic ion exchange resin or ferric chloride in an alcohol such as methanol, ethanol or propanol or in a reaction solvent such as ethyl acetate, tetrahydrofuran or t-butyl ether. The reaction is carried out at a temperature of 0°C to 40°C.

### (Step F)

This step is a step of converting the alcohol derivative (1-7) into the thiol derivative (1-8). The reaction can be carried out in the same manner as described in JP-A 11-35556.
① The alcohol derivative (1-7) is reacted with methane sulfonyl chloride, whereby one of the hydroxyl groups in (1-7) can be converted selectively into a methanesulfonyloxy group. The reaction is carried out in the presence of a base such as triethylamine or pyridine. The reaction can be carried at 0°C to room temperature in a solvent such as tetrahydrofuran, dichloromethane, chloroform, toluene, hexane or diethyl ether.
② Then, the methanesulfonyloxy group is converted into an acylthio group. The reaction is carried out by reaction with a thiocarboxylate such as potassium thioacetate or sodium thioacetate in a polar solvent such as acetonitrile, acetone, 1,2-dimethyl-2-imidazolidinone (DMI), dimethylacetamide (DMA), dimethylformamide (DMF) or dimethyl sulfoxide (DMSO), or by reaction with a thiocarboxylic acid such as thioacetic acid or thiobenzoic acid in the presence of a base such as potassium carbonate or cesium carbonate.

Then, the resulting acylthio derivative is deprotected by removing both the protecting group on the nitrogen atom and the thiol-protecting group in the presence of an alkali reagent such as sodium hydroxide or in the presence of an inorganic acid such as hydrochloric acid or an organic acid such as trifluoroacetic acid in a reaction solvent, for example an alcohol such as methanol, ethanol or propanol, ortetrahydrofuran or t-butyl methyl ether, whereby the thiol derivative (1-8) can be obtained. The reaction is carried out at a temperature of 0 to 60°C.

The solvent used in the present invention is not particularly limited and may be any solvent insofar as it is usually used in organic synthesis without inhibiting the reaction, and examples of the solvent include lower alcohols such as methanol, ethanol, propanol and butanol, polyalcohols such as ethylene glycol and glycerine, ketones such as acetone, methyl ethyl ketone, diethyl ketone and cyclohexanone, ethers such as diethyl ether, isopropyl ether, tetrahydrofuran, dioxane, 2-methoxyethanol and 1,2-dimethoxyethane, nitriles such as acetonitrile and propionitrile, esters such as methyl acetate, ethyl acetate, isopropyl acetate, butyl acetate and diethyl phthalate, halogenated hydrocarbons such as dichloromethane, chloroform, carbon tetrachloride, 1,2-dichloroethane, trichloroethylene and tetrachloroethylene, aromatics such as benzene, toluene, xylene, monochlorobenzene, nitrobenzene, indene, pyridine, quinoline, collidine, and phenol, hydrocarbons such as pentane, cyclohexane, hexane, heptane, octane, isooctane, petroleum benzene and petroleum ether, amines such as ethanolamine, diethylamine, triethylamine, pyrrolidine, piperidine, piperazine, morpholine, aniline, dimethylaniline, benzylamine and toluidine, amides such as formamide, N-methylpyrrolidone and N,N-dimethylformamide, phosphoric acid amides such as hexamethylphosphoric acid triamide and hexamethylphosphorous acid triamide, water, and other generally used solvents, and these solvents can be used alone or as a mixed solvent of two or more thereof wherein the mixing ratio is not particularly limited.

As described above, the base usable in the present invention is usually not particularly limited and may be any base insofar as it does not inhibit the reaction and is known as a base in organic synthesis, and examples of the base include sodium carbonate, sodium bicarbonate, potassium carbonate, sodium hydride, potassium hydride, t-butoxy patassium, pyridine, dimethylaminopyridine, trimethylamine, triethylamine, N,N-diisopropylethylamine, N-methylmorpholine, N-methylpyrrolidine, N-methylpiperidine, N,N-dimethylaniline, 1,8-diazabicyclo[5,4,0]undec-7-ene (DBU), pyridine, 4-dimethylaminopyridine, picoline, lutidine, quinoline, isoquinoline, sodium hydroxide, potassium hydroxide, lithium hydroxide, butyl lithium, and a sodium or potassium alcolate such as sodium methylate, potassium methylate or sodium ethylate.

As described above, the reducing agent usable in the present invention is not particularly limited and may be any reducing agent insofar as it is usually used in organic synthesis without inhibiting the reaction, and mention is made of catalytic hydrogenating catalysts such as sodium borohydride (NaBH₄), lithium borohydride (LiBH₄), zinc borohydride (Zn(BH₄)₂), tetramethyl ammonium boron triacetoxy hydride (Me₄NBH(OAc)₃), sodium cyano borohydride (NaBH₃CN), potassium trialkyl borohydride (Selectride), lithium triethyl borohydride (Super hydride (LiBHEt₃)), lithium aluminum Hydride (LiAlH₄), diisobutyl aluminum hydride (DIBAL), lithium aluminum t-butoxy hydride (LiAlH(t-BuO)₃), sodium bis(2-methoxyethoxy)aluminum hydride (Red-al), 2,2'-bis(diphenylphosphino)-1,1'-binaphthyl (binap), platinum, palladium, rhodium, ruthenium and nickel.

After the reaction described above is finished, the compound of the present invention can be purified and isolated by usual separation means if necessary (for example, recrystallization, chromatography etc.).

The compound of the present invention can be produced by e.g. methods described in the Examples below. However, these examples are shown for illustrative purposes, and the compound of the present invention is not limited to the following examples.

Hereinafter, the Examples are described.

### Example 1

### 1-(t-butyl)-2-methyl-(2S,4R)-4-[1-(t-butyl)-1,1-dimethylsilyl]-oxytetrahydro-1H-1,2-pyrrole dicarboxylate

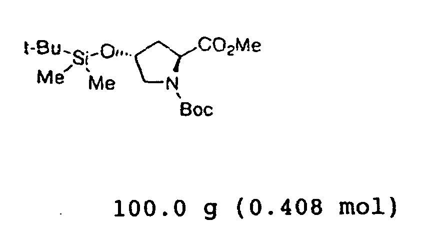

1-(t-butyl)-2-methyl-(2S,4R)-4-hydroxytetrahydro-1H-1,2-pyr role dicarboxylate and 400 mL dimethylformamide (DMF) were introduced into a 1 L flask, and the mixture was completely dissolved at room temperature. 34.70 g (0.510 mol, 1.25 eq) imidazole was added thereto at room temperature, then 76.82 g (0.510 mol, 1.25 eq) t-butyldimethylsilyl chloride (TBSC1) was added thereto under cooling on ice, and the rinse with 100 mL dimethylformamide (DMF) was added thereto (reaction temperature of 8.1°C to 10.9°C). Thereafter, the mixture was stirred at room temperature for 18.5 hours. The reaction mixture was transferred into a 2 L separatory flask and partitioned between 500 mL ethyl acetate (AcOEt) and 500 mL water , and after the aqueous layer was separated, the organic layer was washed with water : methanol (2 : 1), 500 mL (×2) and then saturated brine, 500 mL (×1). The resulting organic layer was dried with 20.0 g magnesium sulfate anhydride and filtered, and the filtrate was concentrated at 40°C under reduced pressure. The concentrated residue was subjected twice to azeotropy with 150 mL toluene to give 145.95 g title compound in a crude form.

### Example 2

### (2S,4R)-N-t-butoxycarbonyl-4-t-butyldimethylsilyloxy-2-formylpyrrolidine

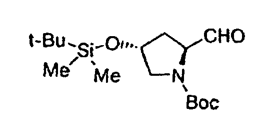

240.35 g (70.3% in toluene, 0.836 mol, 2.05 eq) sodium bis(2-methoxyethoxy)aluminum hydride (Red-Al) and 836 mL t-butyl methyl ether (MTBE) were introduced into a 5-L four-necked flask in a nitrogen atmosphere, and the mixture was stirred and then cooled to -20°C. A solution of 74.30 g (1.045 mmol) pyrrolidine in t-butyl methyl ether (271 mL) was added dropwise thereto over 20 minutes (internal temperature of -20°C to -14.2°C), then the internal temperature was increased to 15°C, and the mixture was stirred for 50 minutes. A solution of 9.38 g (0.0836 mol) potassium t-butoxide in tetrahydrofuran (THF) (42mL) was added dropwise thereto (internal temperature of 18.3°C to 20.4°C), and the mixture was stirred for additional 80 minutes. This reducing agent was cooled to -60°C, and a solution of 145.95 g 1-(t-butyl)-2-methyl-(2S,4R)-4-[1-(t-butyl)-1,1-dimethylsilyl]oxytetrahydro-1H-1,2-pyrrole dicarboxylate (Example 1) in t-butyl methyl ether (510 mL) was added dropwise thereto over 3 minutes (internal temperature of -60.0°C to -55°C), and then the temperature of the reaction solution was increased to about -15°C. The mixture was further stirred for 1 hour and then cooled again to -60°C. 1644 mL of 3 N hydrochloric acid was added dropwise to the reaction solution over 4 minutes (internal temperature of -60.9°C to 4.7°C), and then the reaction was quenched. The reaction solution was transferred to a 5-L separatory flask, and the aqueous layer was separated off and the remain was washed with 1020 mL of 0.1 N hydrochloric acid, 1020 mL of 10% aqueous sodium chloride, 1020 mL of 5% sodium bicarbonate and 1020 mL (2×) of 5% aqueous sodium chloride. The resulting organic layer was dried with 30 g magnesium sulfate anhydride and filtered, and the filtrate was concentrated at 40°C under reduced pressure. The concentrated residue was subjected twice to azeotropy with 500 mL toluene to give 135.5 g crude product containing the title compound.

### Example 3-1

### (3S)-N-t-butoxycarbonyl-3-[[(R)-hydroxy-(2S,4R)-(N-t-butoxycarbonyl-4-t-butyldimethylsilyloxy)pyrrolidin-2-yl]methyl]-pyrrolidin-2-one

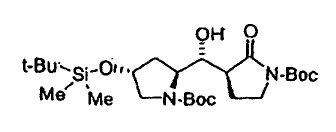

A suspension of cinchonidine (98.2 g, 334 mmol) in tetrahydrofuran (480ml) was cooled to -70°C in a nitrogen stream. 1.0 M lithium bis(trimethylsilyl)amide solution in tetrahydrofuran (668 ml, 668 mmol) was added dropwise to this suspension over 20 minutes. Subsequently, a solution of N-t-butoxycarbonyl-2-oxopyrrolidine (61.8 g, 334 mmol) in tetrahydrofuran (220 ml) was added dropwise thereto over 15 minutes. This solution was stirred at -70°C for 1.5 hours to form an enolate solution.

The prepared enolate solution at -70°C was added dropwise over 15 minutes in a nitrogen stream to a solution of (2S,4R)-N-t-butoxycarbonyl-4-t-butyldimethylsilyloxy-2-formylpyrrolidine (100 mg, 304 mmol) (Example 2) in tetrahydrofuran (500 ml) cooled to -70°C. Thereafter, the temperature of this reaction solution was increased to -40°C, and the solution was stirred for 2.0 hours.

The reaction solution at -40°C was added dropwise over 15 minutes to 40% aqueous citric acid (1000 ml) under cooling on ice, and the temperature of the solution was increased to room temperature. The reaction solution was extracted with ethyl acetate (1000 ml), and the organic layer was washed with 20% aqueous citric acid (1000 ml)×2, water (500 ml), a saturated sodium bicarbonate solution (1000 ml) and water (500 ml) in this order. The organic layer was concentrated by azeotropy with dimethoxyethane, to give a crude product (211 g) containing the title compound. This compound was used in the subsequent reaction without purification.

By high performance liquid chromatography, it was confirmed that this crude product contained the desired compound in step C and 3 diastereomers in a relative area ratio (desired compound : diastereomer 1 : diastereomer 2 : diastereomer 3) of 60 : 24 : 16 : 0.

¹H-NMR (CDCl₃) δ: 0.08 (6H, s), 0.87 (9H, s), 1.45 (9H, s), 1.54 (9H, s), 1.60-1.74 (1H, m), 1.84-2.13 (2H, m), 2.24 (1H, td, J=6, 13Hz), 2.40 (1H, dd, J=11, 21Hz), 3.31 (1H, dd, J=4, 11Hz), 3.39-3.60 (2H, m), 3.76-3.98 (2H, m), 4.13-4.55 (3H, m).

(3S)-N-t-butoxycarbonyl-3-[[(R)-hydroxy-(2S,4R)-(N-t-butoxycarbonyl-4-t-butyldimethylsilyloxy)pyrrolidin-2-yl]me thyl]pyrrolidin-2-one and diastereomers thereof could be obtained in the following selectivity in the same manner as in Example 3-1 except that R¹ and the asymmetric auxiliary were changed.

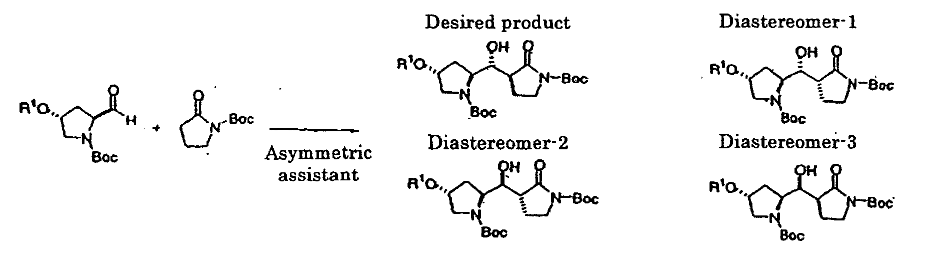

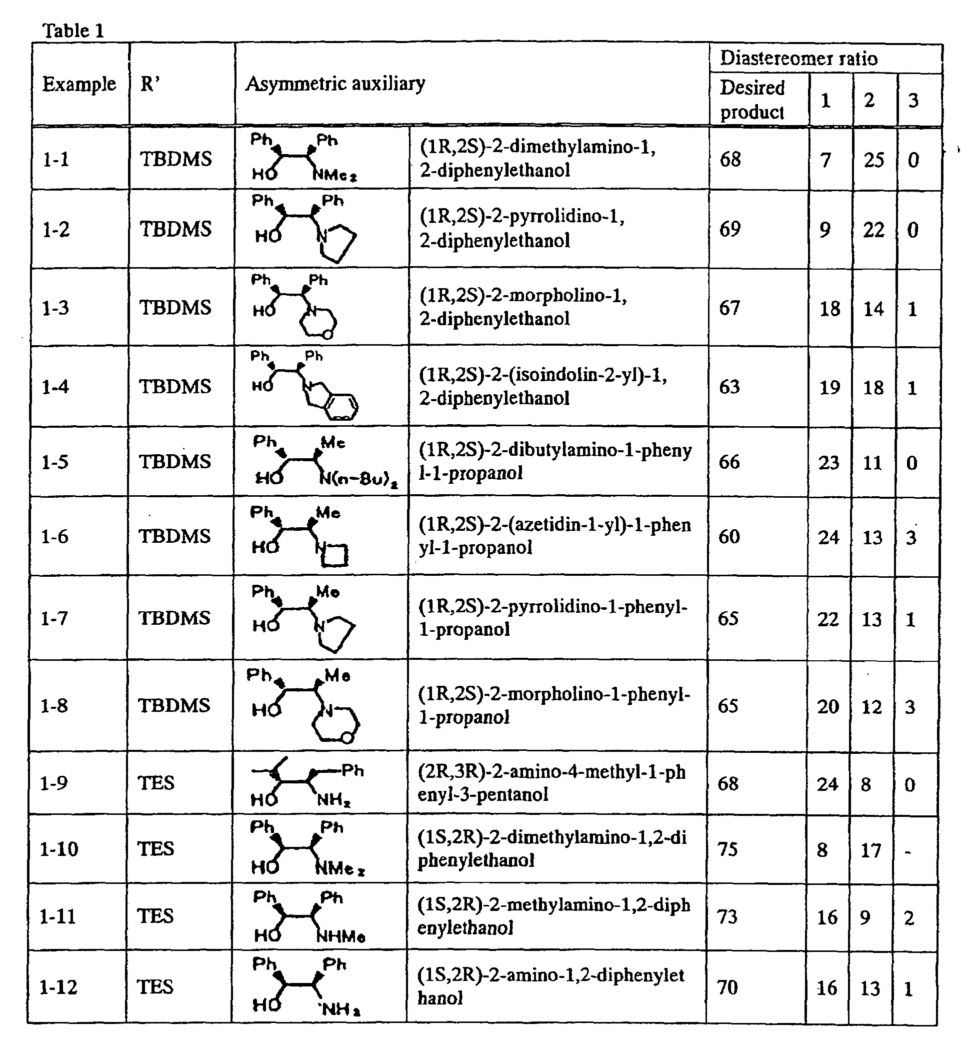

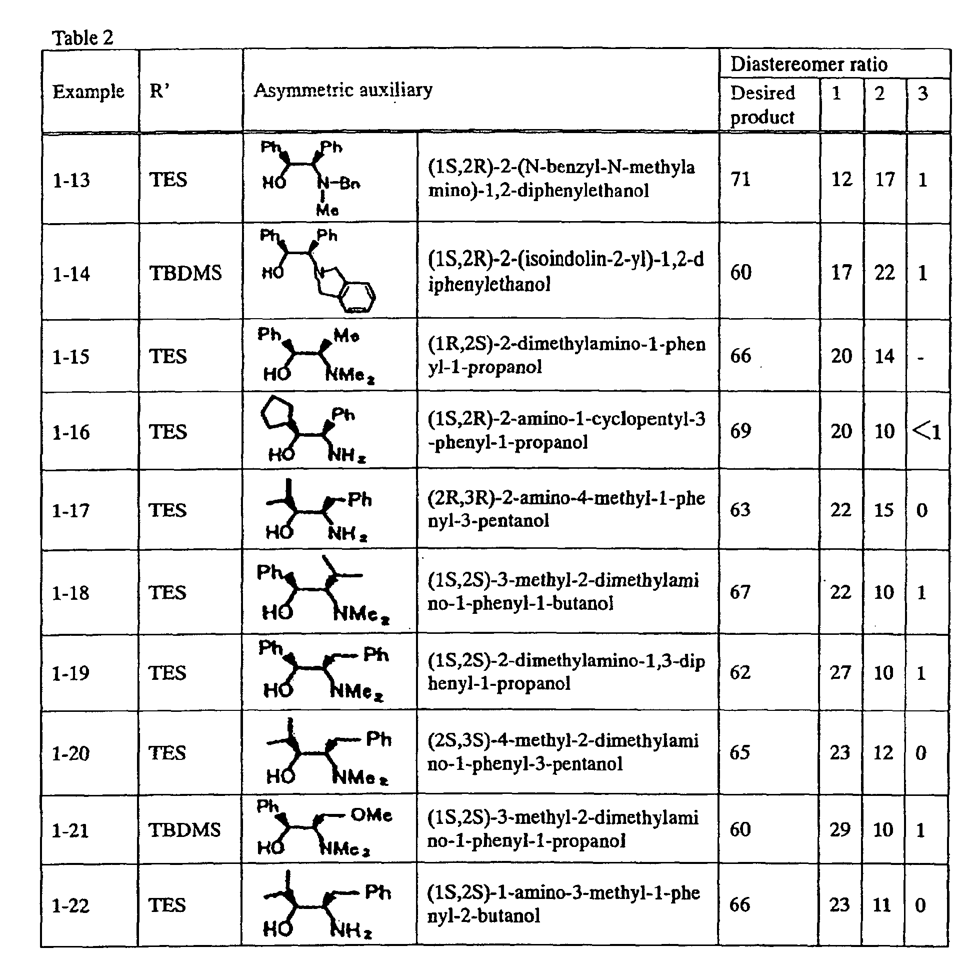

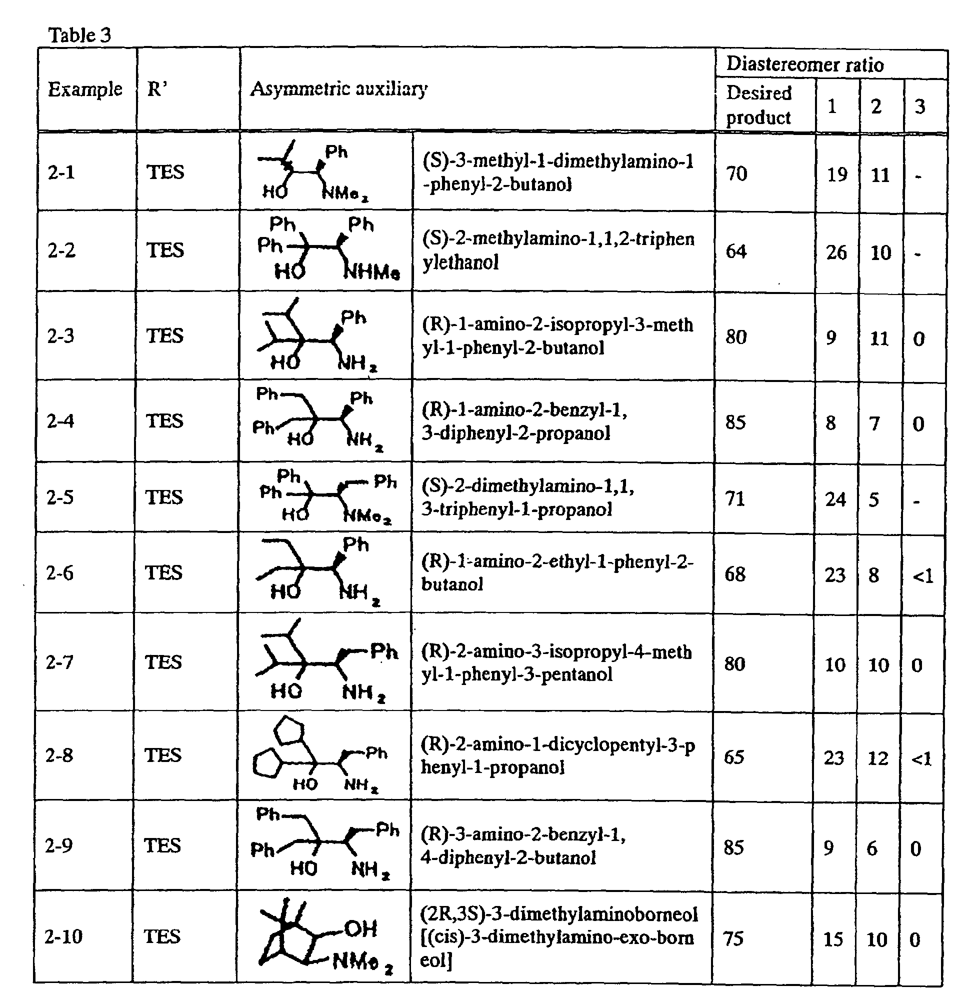

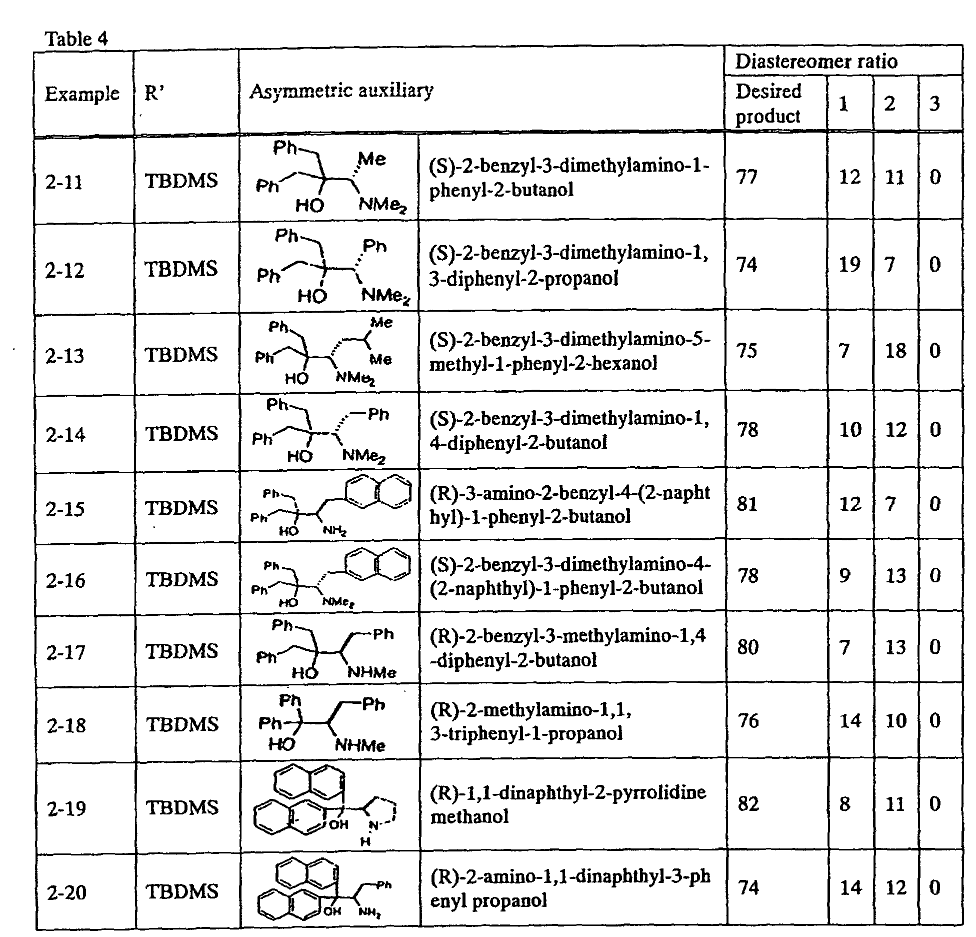

In Tables 1 to 4, TES means a triethylsilyl group, and TBDMS means a t-butyldimethyl silyl group.

### Example 4

### (2S,4R)-N-t-butoxycarbonyl-2-[[(3R)-N-t-butoxycarbonylpyrrolidine-3-yl-(R)-hydroxy]methyl]-4-t-butyldimethylsilyloxypyrrolidine

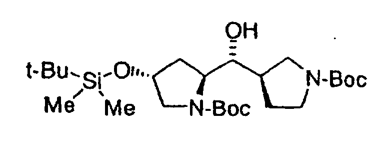

A solution of sulfuric acid (18.1 ml) in dimethoxyethane (91 ml) was added dropwise over 15 minutes to a suspension of sodium borohydride (24.7 g, 652 mmol) in dimethoxyethane (400 ml) under cooling on ice. To this suspension was added dropwise a solution of the crude product (105.5 g) containing (3S)-N-t-butoxycarbonyl-3-[[(R)-hydroxy-(2S, 4R)-(N-t-butoxycarbonyl-4-t-butyldimethylsilyloxy)pyrrolidin-2-yl] methyl] pyrrolidin-2-one (40.9 g, 79.5 mmol) (Example 3-1) in diethoxyethane (240 ml) over 15 minutes under cooling on ice. This reaction solution was stirred at 60°C for 1 hour and quenched with methanol (53 ml) under cooling on ice. Subsequently, this reaction solution was added to 2 N aqueous sodium hydroxide (240 ml) and extracted at 40°C. This organic layer was washed with 10% brine (160 ml)×3. The organic layer was concentrated to give a crude product (71.0 g) containing the title compound. This reaction product was used in the subsequent reaction without purification.

### Example 5-1

### (2S,4R)-N-t-butoxycarbonyl-2-[[(3R)-N-t-butoxycarbonylpyrrolidin-3-yl-(R)-hydroxy]methyl]-4-hydroxypyrrolidine

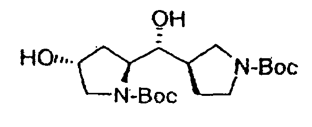

75 wt% aqueous tetrabutyl ammonium fluoride (51.5 ml, 141 mmol) was added dropwise at room temperature to a solution of the crude product (71.0 g) containing (2S,4R)-N-t-butoxycarbonyl-2-[[(3R)-N-t-butoxycarbonylpyrrolidin-3-yl-(R)-hydroxy]methyl]-4-t-butyldimethylsilyloxypyrrolidine (Example 4) in tetrahydrofuran (120 ml). This reaction solution was stirred at 40°C for 2.5 hours, then diluted with 75 wt% aqueous tetrabutyl [sic] ammonium fluoride (240 ml) and extractedwith t-butyl methyl ether (240 ml). This organic layer was washed with 10% aqueous citric acid (120 ml)×3, 5% brine (60 ml), 5% aqueous sodium bicarbonate (120 ml) and water (60 ml)×2 in this order. The organic layer was concentrated, and the residues (52.3 g) were concentrated under azeotropy with n-butyl acetate, then n-butyl acetate (192 ml) and n-heptane (385 ml) were added thereto, and the mixture was dissolved by heating at 60°C. The solution was cooled gradually from 60°C to 23°C and stirred for 16 hours. The precipitated crystals were filtered off under suction and washed with n-butyl acetate/n-heptane (1 : 2) (96 ml). The crystals were dried under reduced pressure to give crystals of the title compound (15.3 g; content, 96%).

By high performance liquid chromatography, it was confirmed that the product contained the title compound and 3 diastereomers in a relative area ratio (title compound : diastereomer 1 : diastereomer 2 : diastereomer 3) of 95 : 3.3 : 1.6 : 0.
¹H-NMR (CDCl₃) : 1.46 (s, 9H), 1.48 (s, 9H), 1.65-2.15 (m, 5H), 3.10-3.40 (m, 3H), 3.41-3.80 (m, 3H), 3.96-4.20 (m, 2H), 4.44 (brs, 1H)

### Example 5-2

### (2S,4R)-N-t-butoxycarbonyl-2-[[(3R)-N-t-butoxycarbonylpyrrolidin-3-yl-(R)-hydroxy]methyl]-4-hydroxypyrrolidine (alternative method)

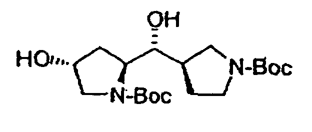

0.37 g (1.1 mmol) of 10% hydrochloric acid, 0.5 g (1.0 mmol) (3S)-N-t-butoxycarbonyl-3-[[(3R)-hydroxy-(2S,4R)-t-butoxyca rbonyl-4-butyldimethylsilyloxy]pyrrolidin-2-yl]methyl]pyrro lidine (Example 4) and 0.8 ml methanol were mixed and reacted at 20°C for 1 hour. Then, the reaction solution at an internal temperature of 0°C or less was added dropwise to 20 ml of 7% aqueous sodium bicarbonate and then extracted with 10 ml t-butyl methyl ether. The solvent in the organic layer was distilled awayunder reduced pressure, and the residues were recrystallized from 3.8 g n-butyl acetate/8.9 g n-pentane to give 0.33 g of the title compound (yield, 85%).

### Example 5-3

### (2S,4R)-N-t-butoxycarbonyl-2-[[(3R)-N-t-butoxycarbonylpyrrolidin-3-yl-(R)-hydroxy]methyl]-4-hydroxypyrrolidine (alternative method)

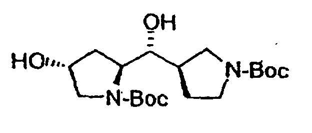

0.29 g (3 mmol) phosphoric acid, 0.5 g (1.0 mmol) (3S)-N-t-butoxy-(2S,4R)-(N-t-butoxycarbonyl-4-hydroxy) pyrrolidin-2-yl) methyl) pyrrolidine (Example 4), 2 ml tetrahydrofuran and 1 ml water were mixed and reacted at 50°C for 8 hours. The reaction solution was added dropwise to 20 ml of 5% aqueous sodium bicarbonate under stirring and then extracted with 30 ml t-butyl methyl ether. The solvent in the organic layer was distilled away under reduced pressure, and then the resulting residues were recrystallized from 3.5 g n-butyl acetate/8.2 g n-pentane to give 0.32 g of the title compound (yield, 83.2%).

### Example 5-4

### (2S,4R)-N-t-butoxycarbonyl-2-[[(3R)-N-t-butoxycarbonylpyrrolidin-3-yl-(R)-hydroxy]methyl]-4-hydroxypyrrolidine (alternative method)

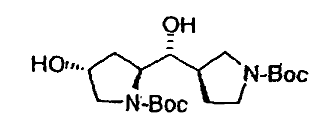

0. 36 g (1.7 mmol) ferric chloride·6H₂O, 0.5 g (1.0 mmol) (3S)-N-t-butoxy-(2S,4R)-(N-t-butoxycarbonyl-4-hydroxy) pyrrolidin-2-yl] methyl] pyrrolidine (Example 4), 2 ml tetrahydrofuran and 1 ml water were mixed and reacted at 20°C for 5 hours. The reaction solution was added dropwise to 20 ml of 5% aqueous sodium bicarbonate under stirring and then extracted with 30 ml t-butyl methyl ether. The solvent in the organic layer was distilled away under reduced pressure, and the resulting crude product was recrystallized from 3.5 g n-butyl acetate/8.2 g n-pentane to give 0.32 g of the title compound (yield, 83.2%).

The optically active amino-alcohol can be synthesized in a generally used method by reacting an optically active amino acid or a protected optically active amino acid with an organometallic reagent such as an organolithium reagent or an organomagnesium reagent. Specifically, various optically active alcohols can be synthesized according to methods described in JP-A 56-65848, Chem. Pharm. Bull. (1969) p. 145, or Tetra. Lett. (1990) p. 4985, etc. Some methods of producing the amino-alcohol as the asymmetric auxiliary used in the present invention are specifically as follows.

### Reference Example 1

### (R)-1-amino-2-benzyl-1,3-diphenyl-2-propanol

83.2 g d-phenylalanine methyl ester benzophenone imine was dissolved in 250 ml tetrahydrofuran and flushed 3 times with nitrogen. 455 ml benzyl magnesium chloride (1.06 M in tetrahydrofuran) was added dropwise thereto under cooling on ice (internal temperature of 4 to 19°C), and the mixture was reacted as such at room temperature for 2 hours. After the disappearance of the starting material was confirmed by TLC, the reaction solution was cooled again to 0°C and quenched with 230 ml saturated ammonium chloride. 230 ml water was added to the reaction solution which was then extracted with 450 ml ethyl acetate, and the organic layer was washed with 450 ml of 10% sodium chloride and concentrated to give 128.1 g addition product. This product was dissolved in 620 ml tetrahydrofuran, then 310 ml of 2 N hydrochloric acid was added thereto, and the mixture was reacted for 2 hours in an oil bath at 50 to 60°C. After the disappearance of the starting material was confirmed, the reaction solution was returned to room temperature and then poured into a separatory funnel containing 1240 ml hexane and 310 ml water. The rinse with 190 ml methanol was added thereto. After extraction and separation, 360 ml of 2 N sodium hydroxide and 930 ml ethyl acetate (aqueous layer, pH 8) were added thereto, and after extraction and separation the organic layer was washed with 450 ml of 10% sodium chloride, dried with 100 g sodium sulfate, filtered and concentrated to give 63.4 g crude crystals. 650 ml t-butyl methyl ether was added to the crude crystals which were then dissolved under reflux, and the solution was left and cooled to room temperature to form crystals. 1300 ml hexane was added to the crystals, cooled to 0°C and stirred for 1 hour, and the crystals were filtered off. The crystals were washed with (cold) hexane/t-butyl methyl ether (3:1) and dried under reduced pressure to give 41.3 g of the title compound ((R)-1-amino-2-benzyl-1,3-diphenyl-2-propanol).

### Reference Example 2

### (R)-1-amino-2-benzyl-1,3-diphenyl-2-propanol (alternative method)

300 g (1.39 mol) D-phenylalanine methyl ester hydrochloride was suspended in 3 L tetrahydrofuran and flushed 3 times with nitrogen. 2.78 L (eq) benzyl magnesium chloride (2.0 M in tetrahydrofuran) was added dropwise thereto over 90 minutes under cooling on ice (internal temperature of 3 to 15°C). 75 minutes after this dropwise addition, the mixture was quenched (internal temperature of 1 to 31°C) with 4.8 L of 10% ammonium chloride, while paying attention to evolution of heat. The reaction solution was transferred to a 20-L separatory funnel and then extracted with 6 L ethyl acetate, but because of poor separation, 3 L water was added thereto. After separation, the organic layer was washed with 3 L of 10% ammonium chloride, 3 L of 18% sodium chloride, and 3 L of 7% sodium chloride in this order. 500 g magnesium sulfate was added to the solution which was then dried, filtered and concentrated, and the resulting crude crystals were recrystallized from 2.5 L t-butyl methyl ether to give 238 g of the title compound

### ((R)-1-amino-2-benzyl-1,3-diphenyl-2-propanol).

### Reference Example 3

### (R)-2-benzyl-3-methylamino-1,4-diphenyl-2-butanol

40.0 g (120.7 mmol) (R)-1-amino-2-benzyl-1,3-diphenyl-2-propanol and 96 mL formamide were introduced into a 300-mL flask and the resulting suspension was heated. The material was dissolved completely at 170°C and stirred as such for 3.5 hours. After completion of the reaction was confirmed by NH silica thin layer chromatography (ethyl acetate : hexane = 1 : 1), the reaction solution was cooled to room temperature and partitioned between 500 mL ethyl acetate and 100 mL water and then washed with 50 mL water and 50 mL saturated sodium chloride. The organic layer was dried with magnesium sulfate anhydride and filtered, and the filtrate was concentrated under reduced pressure to give 62.48 g concentrate. The concentrated residue was purified in the from of a suspension under heating at 90°C by adding 60 mL t-butyl methyl ether and 110 ml ethyl acetate. 110 mL solvent was distilled away, and the resulting crystals were filtered off under cooling on ice. The resulting crystals were washed with 40 mL cold t-butyl methyl ether and dried at room temperature for 1 hour to give 32.05 g (89.16 mmol; yield 74%) N-formamide derivative. 84.0 g sodium bis(2-methoxyethoxy)aluminum hydride (Red-Al) (70% in Toluene, 292.1 mmol, 3.5 eq) and 84 mL tetrahydrofuran were introduced into a 2-L four-necked flask in a nitrogen atmosphere. A solution of 30.00 g (83.46 mmol) formyl derivative in 600 mL tetrahydrofuran was slowly added dropwise thereto under cooling on ice. After this addition, the mixture was heated for 1.5 hours under reflux, and after the completion of the reaction was confirmed by NH silica thin layer chromatography (ethyl acetate : hexane = 1 : 1), the reaction was quenched with 293 mL of 4 N sodium hydroxide under cooling on ice. 1000 mL t-butyl methyl ether was added thereto, after separation, and the organic layer was washed with 100 mL of 2 N sodium hydroxide, 100 mL water and 150 mL saturated sodium chloride in this order. The organic layer was dried with sodium sulfate anhydride and then filtered, and the filtrate was concentrated under reduced pressure to give 30.32 g concentrate. The concentrated residue was crystallized from 75 mL t-butyl methyl ether to give 20.82 g of the title compound ((R)-2-benzyl-3-methylamino-1,4-diphenyl-2-butanol).

### Reference Example 4

### (1S,2S)-2-dimethylamino-1,3-diphenyl-1-propanol

2.0 g (5.6 mmol) d-phenylalanine ethyl ester benzophenone imine was dissolved in 60 ml dichloromethane, and a mixture of 5.6 ml DIBAL (1 M in hexane) and 5.6 ml triisobutylaluminum (TRIBAL) (1 M in hexane) was added dropwise thereto at -78°C. After the mixture was stirred at -78°C for 30 minutes, 5.6 ml phenyl magnesium bromide (PhMgBr) (3 M in ether) was added dropwise thereto, and the mixture was reacted while the temperature of the mixture was increased to room temperature. After 1 hour, the reaction mixture was cooled again and quenched at -30°C with saturated ammonium chloride (sat. NH₄Cl). The resulting gel was dissolved and separated with 2 N hydrochloric acid, and the organic layer was washed with 10% sodium chloride -5% sodium bicarbonate and then concentrated. The resulting crude product was purified by silica gel (SiO₂) column chromatography (5% ethyl acetate/Hex → 10%) to give 740 mg iminoalcohol. This product was dissolved in 10 ml tetrahydrofuran, then 10 ml of 1 N hydrochloric acid was added thereto, and the mixture was reacted at room temperature for 14 hours. 30 ml hexane was added to the reaction solution. After washing and separation, it was adjusted to pH 9 with 2 N sodium hydroxide and extracted with ethyl acetate, and the solvent was distilled away, whereby 390 mg amino-alcohol was obtained. 1. 2 ml of 38% formaldehyde, 1.2 ml formic acid and 1.2 ml acetonitrile were added thereto and stirred for 14 hours under heating at 70°C. The reaction solution was diluted with water, adjusted to pH 9 with 2 N sodium hydroxide and extracted with ethyl acetate. The solvent was concentrated, and the resulting crude product was purified by NH SiO₂ column chromatography (10% ethyl acetate/Hex) to give 360 mg of the title compound ((1S,2S)-2-dimethylamino-1,3-diphenyl-1-propanol).

In the foregoing description, the term "eq" refers to equivalent, "Hex" to hexane, "pH" to hydroxide ion concentration, and "A" in the terms "in A" to a solvent.

### INDUSTRIAL APPLICABILITY

The effect of the present invention is compared with that of the prior art. In an invention described in JP-A 8-73462, unnecessary diastereomers of ((3S)-3-[[(R)-hydroxy-(2S,4R)-(N-t-butoxycarbonyl-4-alkylsi lyloxy) pyrrolidin-2-yl]methyl]pyrrolidin-2-one derivative) are obtained in a larger amount, and thus the yield of the compound of this compound was a low yield of 30.2%.

In an invention described in JP-A 11-35556, the above compound ((3S)-3-[[(R)-hydroxy-(2S,4R)-(N-t-butoxycarbonyl-4-alkylsi lyloxy) pyrrolidin-2-yl]methyl]pyrrolidin-2-one derivative) was obtained in a high yield of 63 to 67%, but in this production method (Evans method), there was disadvantageous in that the compound should be produced via expensive asymmetric oxazolidine and a highly explosive azide derivative.

In the present invention, asymmetric aminoethanol was used as a catalyst, and as a result, the above compound can be obtained inexpensively and safely in a high yield of 60 to 85% with a smaller number of steps, and there could be provided a production process for synthesis in a large amount.

In the present invention, the above compound is converted into the desired compound via an intermediate ((2S,4R)-N-t-butoxycarbonyl-2-[[(3R)-N-t-butoxycarbonylpyrr olidin-3-yl-(R)-hydroxy]methyl]-4-hydroxypyrrolidine), and this intermediate ((2S,4R)-N-t-butoxycarbonyl-2-[[(3R)-N-t-butoxycarbonylpyrr olidin-3-yl-(R)-hydroxy]methyl]-4-hydroxypyrrolidine) has good crystallizability and can thus be easily separated and purified. By virtue of the two advantages, there brings about an effect of significantly increasing the yield of (2S,4S)-2-[[(3R)-pyrrolidin-3-yl-(R)-hydroxy]methyl]pyrroli din-4-thiol dihydrochloride.

In addition, the aminoethanol used as an asymmetric assistant can be easily separated by a partition procedure, is generally excellent in crystallizability, and can thus be purified and reutilized to make the process more economical.

## Claims

1. A process for producing an optically active pyrrolidine-4-thiol derivative (IX) represented by the following formula, or salts thereof or hydrates thereof: 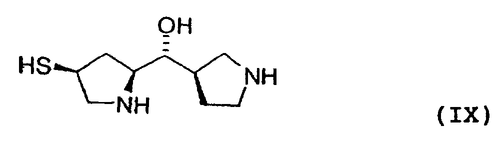 which comprises introducing a protecting group into a hydroxyl group of a pyrrolidine derivative (I) represented by the formula: 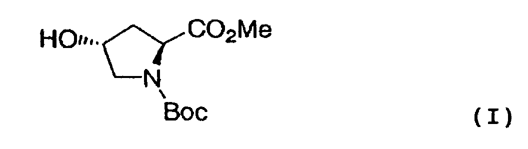 wherein Boc represents a t-butoxycarbonyl group, to form a pyrrolidine-2-carboxylate derivative (II) represented by the formula: 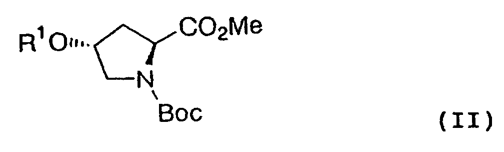 wherein R¹ represents a hydrogen atom or a tri(C₁₋₆ alkyl)silyl group, and Boc has the same meaning as defined above,
then reducing an alkoxycarbonyl group of the compound (II) to give a pyrrolidine-2-carbaldehyde (III) represented by the formula: 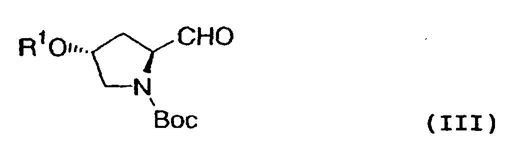 wherein R¹ and Boc have the same meaning as defined above,
then subjecting the compound (III) to asymmetric aldol reaction with a pyrrolidone derivative (V) represented by the formula: 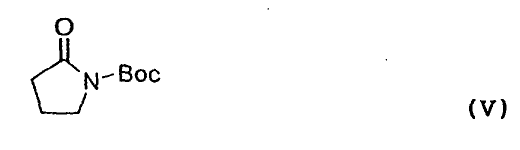 wherein Boc has the same meaning as defined above, by using as an asymmetric assistant an optically active aminoethanol derivative (IVa) represented by the formula: 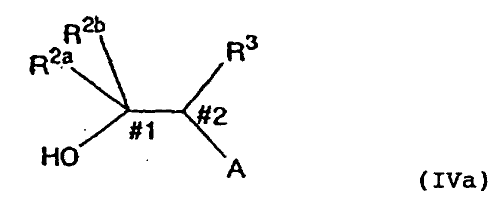 wherein both / either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group) , an 1-pyrrolidinyl group, a 4-morpholinyl group or a 2-isoindolinyl group, an optically active pyrrolidine derivative (IVb) represented by the formula: 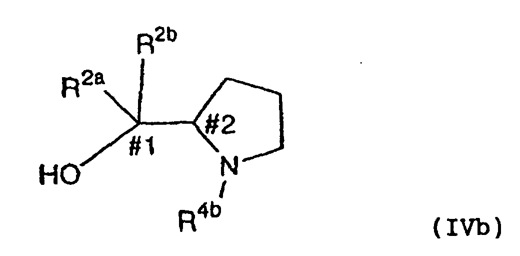 wherein #1, #2, R^{2a}, R^{2b} and R^{4b} have the same meaning as defined above, optically active cinchonidine and/or (2R,3S)-3-dimethylaminoborneol, thereby forming an optically active pyrrolidine derivative (VI) represented by the formula: 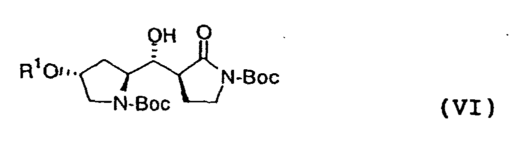 wherein R¹ and Boc have the same meaning as defined above,
then reducing the optically active pyrrolidine derivative (VI) to form a pyrrolidine derivative (VII) represented by the formula: 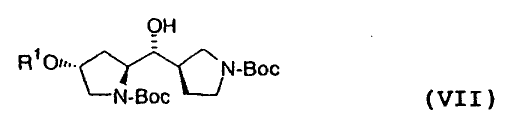 wherein R¹ and Boc have the same meaning as defined above,
then selectively eliminating an alkylsilyl group from the compound (VII) to form an optically active 4-hydroxypyrrolidine derivative (VIII) represented by the formula: 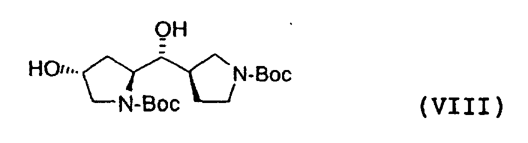 wherein Boc has the same meaning as defined above, and
then converting a hydroxyl group of the compound (VIII) into an acylthio group, to eliminate the protecting group,
whereby the optically active pyrrolidine-4-thiol derivative (IX), or salts thereof or hydrates thereof are obtained.

2. A process for producing an optically active pyrrolidine derivative (VI) represented by the following formula, or hydrates thereof: 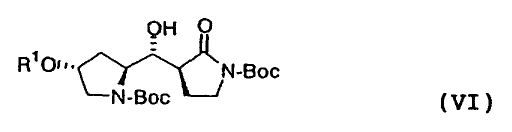 wherein R¹ has the same meaning as defined above, and Boc represents a t-butoxycarbonyl group,
which comprises introducing a protecting group into a hydroxyl group of a pyrrolidine derivative (I) represented by the formula: 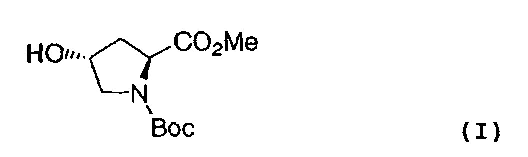 wherein Boc has the same meaning as defined above, to form a pyrrolidine-2-carboxylate derivative (II) represented by the formula: 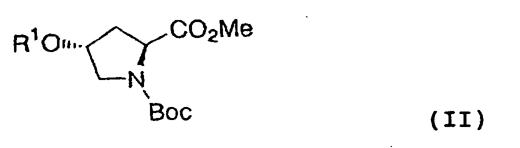 wherein R¹ and Boc have the same meaning as defined above,
then reducing an alkoxycarbonyl group of the compound (II) to give a pyrrolidine-2-carbaldehyde derivative (III) represented by the formula: 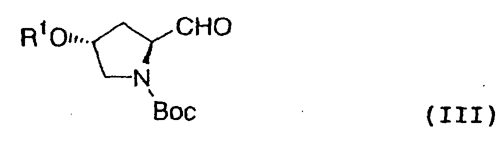 wherein R¹ and Boc have the same meaning as defined above, and
then subjecting the compound (III) to asymmetric aldol reaction with a pyrrolidone derivative (V) represented by the formula: 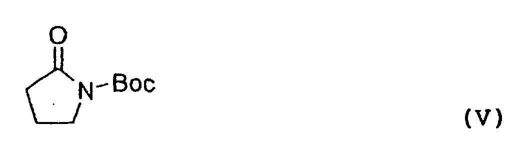 wherein Boc has the same meaning as defined above, by using as an asymmetric assistant an optically active aminoethanol derivative (IVa) represented by the formula: 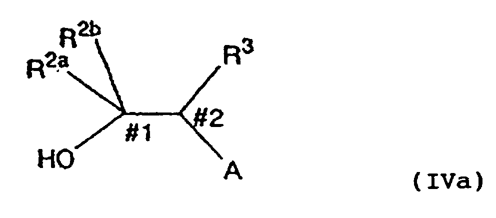 wherein both / either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group), an 1-pyrrolidinyl group, a 4-morpholinyl group or a 2-isoindolinyl group, an optically active pyrrolidine derivative (IVb) represented by the formula: 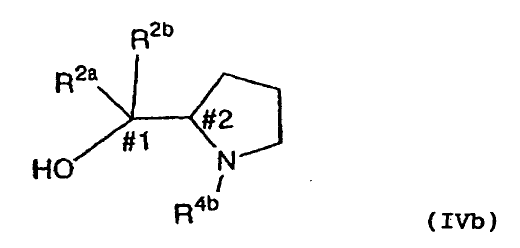 wherein #1, #2, R^{2a}, R^{2b} and R^{4b} have the same meaning as defined above, optically active cinchonidine and/or (2R,3S)-3-dimethylaminoborneol,
whereby the optically active pyrrolidine derivative (VI) or hydrates thereof are obtained.

3. A process for producing an optically active pyrrolidine-4-thiol derivative (IX) represented by the following formula, or salts thereof or hydrates thereof: 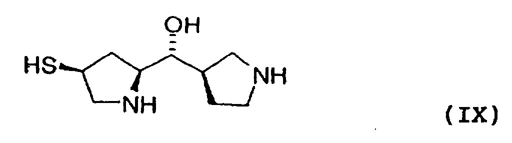 which comprises reducing an optically active pyrrolidine derivative (VI) represented by the formula: 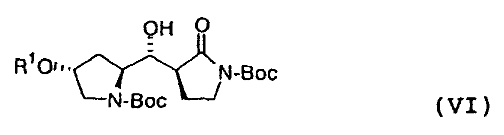 wherein R¹ and Boc have the same meaning as defined above, to form a pyrrolidine derivative (VII) represented by the formula: 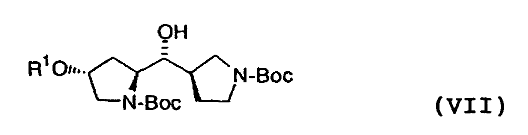 wherein R¹ and Boc have the same meaning as defined above,
then selectively eliminating an alkylsilyl group from the compound (VII), to form an optically active 4-hydroxypyrrolidine derivative (VIII) represented by the formula: 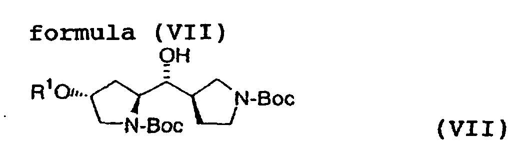 wherein Boc has the same meaning as defined above, and
then converting a hydroxyl group of the compound (VIII) into an acylthio group to eliminate the protective group,
whereby the optically active pyrrolidine-4-thiol derivative (IX), or salts thereof or hydrates thereof are obtained.

4. A process for producing an optically active 4-hydroxypyrrolidine derivative (VIII) represented by the following formula, or hydrates thereof: 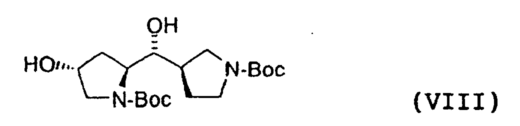 wherein Boc has the same meaning as defined above [not defined] ,
which comprises subjecting a pyrrolidone compound (V) represented by the formula: 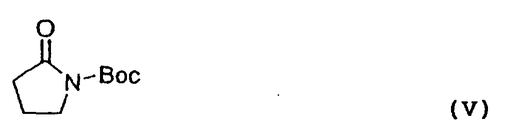 wherein Boc has the same meaning as defined above, to asymmetric aldol reaction with a pyrrolidine-2-carbaldehyde derivative (III) represented by the formula: 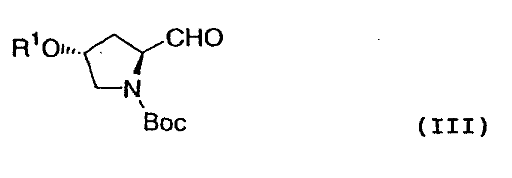 wherein R¹ and Boc have the same meaning as defined above, by using as an asymmetric assistant an optically active aminoethanol derivative (IVa) represented by the formula: 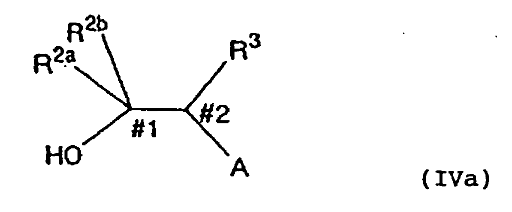 wherein both / either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group), an 1-pyrrolidinyl group, a 4-morpholinyl group or a 2-isoindolinyl group, an optically active pyrrolidine derivative (IVb) represented by the formula: 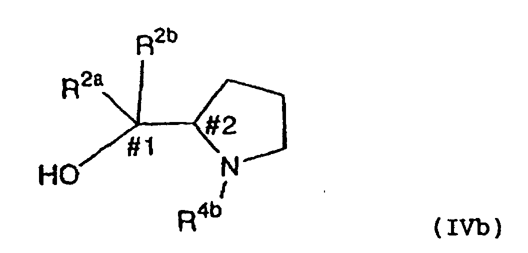 wherein #1, #2, R^{2a}, R^{2b} and R^{4b} have the same meaning as defined above, optically active cinchonidine and/or
(2R,3S)-3-dimethylaminoborneol, thus obtaining an optically active pyrrolidine derivative (VI) represented by the formula (VI): 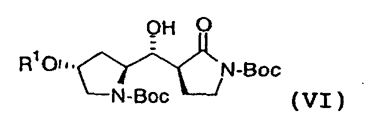 wherein R¹ and Boc have the same meaning as defined above,
then reducing the optically active pyrrolidine derivative (VI) to form a pyrrolidine derivative (VII) represented by the formula: 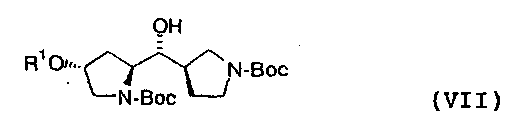 wherein R¹ and Boc have the same meaning as defined above, and
then selectively eliminating an alkylsilyl group from the compound (VII),
whereby the optically active 4-hydroxypyrrolidine derivative (VIII) or hydrates thereof are obtained.

5. A process for producing an optically active pyrrolidine derivative (VI) represented by the following formula, or hydrates thereof: 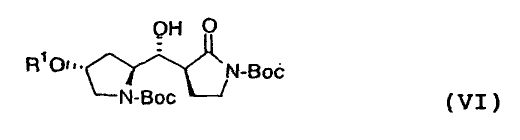 wherein R¹ and Boc have the same meaning as defined above,
which comprises subjecting a pyrrolidine derivative (V) represented by the formula: 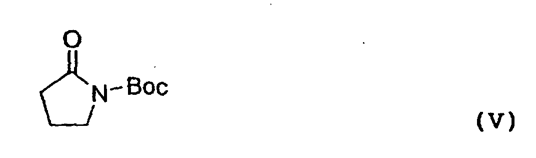 wherein Boc has the same meaning as defined above, to asymmetric aldol reaction with a pyrrolidine-2-carbaldehyde derivative (III) represented by the formula: 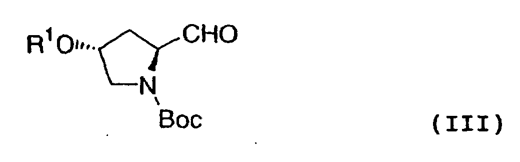 wherein R¹ and Boc have the same meaning as defined above, by using an optically active aminoethanol derivative (IVa) represented by the formula: 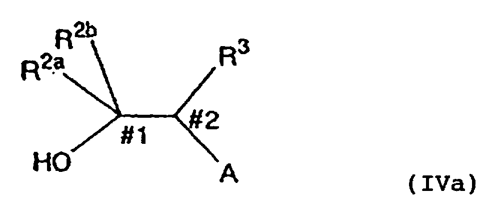 wherein both / either the steric configuration of substituent groups around a carbon atom indicated by #2 and/or the steric configuration of substituent groups around a carbon atom indicated by #1 in the case where R^{2a} and R^{2b} are not the same are / is an R or S conformation, thus indicating that the compound (IVa) is optically active; R^{2a}, R^{2b} and R³ are the same or different and each represent a hydrogen atom, a C₁₋₆ alkyl group, a C₃₋₈ cycloalkyl group, a C₆₋₁₄ aryl group, a C₆₋₁₄ aryl-C₁₋₆ alkyl group or a methoxymethyl group; A is a group represented by formula -NR^{4a}R^{4b} (R^{4a} and R^{4b} are the same or different and each represent a hydrogen atom, a C₁₋₆ alkyl group, a C₃₋₈ cycloalkyl group, a C₆₋₁₄ aryl group or a C₆₋₁₄ aryl-C₁₋₆ alkyl group), an 1-pyrrolidinyl group, a 4-morpholinyl group or a 2-isoindolinyl group, an optically active pyrrolidine derivative (IVb) represented by the formula: 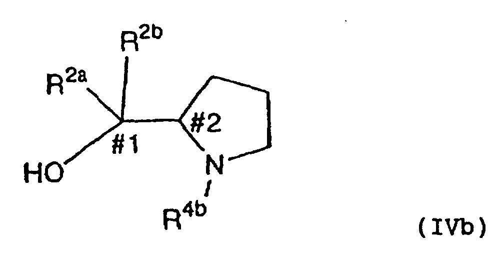 wherein #1, #2, R^{2a}, R^{2b} and R^{4b} have the same meaning as defined above, optically active cinchonidine and/or (2R,3S)-3-dimethylaminoborneol,
whereby the optically active pyrrolidine derivative (VI) or hydrates thereof are obtained.

6. A process for producing an optically active 4-hydroxypyrrolidine derivative (VIII) represented by the following formula, or hydrates thereof: 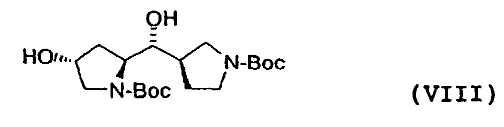 wherein Boc has the same meaning as defined above, which comprises selectively eliminating an alkylsilyl group from a pyrrolidine derivative (VII) represented by the formula: 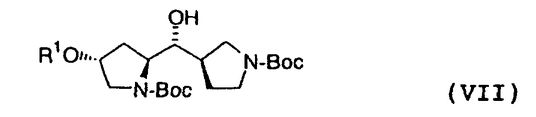 wherein R¹ and Boc have the same meaning as defined above.

7. The process according to any one of claims 1, 2, 4 and 5, wherein the optically active asymmetric assistant is (R)-1-amino-2-benzyl-1,3-diphenyl-2-propanol.

8. The process according to any one of claims 1, 2, 4 and 5, wherein the optically active asymmetric assistant is (R)-3-amino-2-benzyl-1,4-diphenyl-2-butanol.

9. An optically active pyrrolidine derivative (VII) represented by the following formula, or hydrates thereof: 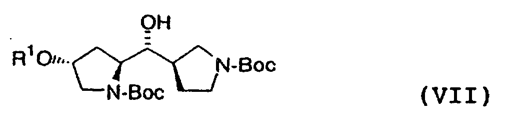 wherein R¹ and Boc have the same meaning as defined above.
